(19) Europäisches Patentamt
European Patent Office
Office européen des brevets

(11) Veröffentlichungsnummer : **0 075 174**
**B1**

(12) # EUROPÄISCHE PATENTSCHRIFT

(45) Veröffentlichungstag der Patentschrift : **07.08.85**

(51) Int. Cl.[4] : **C 07 C 85/04**, C 07 C 87/60

(21) Anmeldenummer : **82108191.6**

(22) Anmeldetag : **06.09.82**

(54) **Verfahren zur Herstellung von 4-Nitrodiphenylaminen.**

(30) Priorität : **17.09.81 DE 3137041**

(43) Veröffentlichungstag der Anmeldung : **30.03.83 Patentblatt 83/13**

(45) Bekanntmachung des Hinweises auf die Patenterteilung : **07.08.85 Patentblatt 85/32**

(84) Benannte Vertragsstaaten : **BE DE FR GB IT NL**

(56) Entgegenhaltungen : **FR-A- 2 359 825**

(73) Patentinhaber : **BAYER AG**
**Konzernverwaltung RP Patentabteilung**
**D-5090 Leverkusen 1 Bayerwerk (DE)**

(72) Erfinder : **Heise, Klaus-Peter, Dr.**
**Dünnwalder Weg 32**
**D-5060 Bergisch Gladbach 2 (DE)**
Erfinder : **Wedemeyer, Karlfried, Dr.**
**Bilharzstrasse 7**
**D-5000 Köln 80 (DE)**

**EP 0 075 174 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 4-Nitrodiphenylaminen durch Umsetzung von Halogennitrobenzolen mit primären aromatischen Aminen in Gegenwart von Kupfer oder Kupferverbindungen und in Gegenwart eines Neutralisationsmittels.

Die Umsetzung von Halogennitrobenzolen mit aromatischen Aminen ist bereits sit langem bekannt. So ist aus der DE-PS 185 663, der GB-PS 24 091 und der FR-PS 381 230 bekannt, die Umsetzung in Gegenwart von Alkalicarbonaten und Kupferverbindungen als Katalysatoren durchzuführen.

Außerdem ist bekannt, daß die außerordentlich langsam verlaufende Reaktion beschleunigt werden kann, wenn Kaliumcarbonat eingesetzt und das Reaktionswasser durch azeotrope Destillation entfernt wird. Gemäß Beispiel 1 der US-PS 2 927 943 wurde unter diesen Bedingungen in 21 Stunden Reaktionszeit mäßig reines 4-Nitrodiphenylamin in einer Ausbeute von 73 % der Theorie erhalten. Aus der US-PS 4 155 936 ist weiterhin bekannt, daß bei der Umsetzung von Halogennitrobenzolen mit primären aromatischen Aminen zu dem Nachteil langer Reaktionszeiten die Verunreinigung der Nitrodiphenylamine infolge Bildung nicht unerheblicher Mengen an Teeren und Nebenprodukten kommt, wie die Bildung von Nitrobenzol infolge reduktiver Enthalogenierung (vgl. US-PS 3 313 854, Spalte 3, Zeilen 64, 65).

Um diese Nachteile zu vermeiden, wurden dem Reaktionsgemisch polare Lösungsmittel als Cokatalysatoren zugesetzt. Allerdings sind auch die bislang eingesetzten polaren Lösngsmittel durchweg mit Nachteilen verbunden. So ist das Dimethylformamid, das gemäß der US-PS 3 055 940 eingesetzt wird, bei den Umsetzungsbedingungen flüchtig und bildet schwer abtrennbare Nebenprodukte. Hexamethylphosphorsäuretriamid, gemäß der US-PS 3 055 940 und Formanilid gemäß der US-PS 3 313 854 eingesetzt, besitzen in katalytischen Mengen in Gegenwart von Kupferverbindungen nur unbefriedigend beschleunigende Eigenschaften. Dimethylsulfoxid, Acetanilid sowie Salicylanilid, die gemäß der US-PS 3 277 175, der DE-AS 1 518 307 und der DE-AS 1 117 594 eingesetzt werden, ergeben ebenfalls nur geringe Effekte. Bei Einsatz der genannten polaren Lösungsmittel wie auch bei dem Einsatz von N-Methylpyrrolidon gemäß der DE-OS 2 633 811 oder ε-Caprolactam gemäß J 56022-751 (1981) sind zudem die Verfahren entweder von vornherein auf die Verwendung von Kaliumcarbonat als Neutralisationsmittel beschränkt oder es werden bei Verwendung von Natriumcarbonat (vgl. DE-AS 1 117 594, Beispiele 14 und 15) oder anderen Neutralisationsmitteln (s. DE-AS 1 518 307, Spalte 3, Zeilen 31-34) merklich schlechtere Ausbeuten und Reinheiten an 4-Nitrodiphenylaminen erzielt.

Auch der in der US-PS 4 155 936 beschriebene Zusatz von Polyethern ist mit Nachteilen verbunden. Je nach Aufarbeitungsweise verbleiben nämlich die Zusatzstoffe im Endprodukt oder im Abwasser. Außerdem ist der Umgang mit Polyethern toxikologisch nicht unbedenklich.

In der US-PS 3 121 736 wird als weitere Möglichkeit zur Verminderung des Teeranralls beschrieben der Zusats von Aminocarbonsäuren, wie Glutaminsäure oder Phenylalanin, von Alkyldiaminopolycarbonsäuren und ihren Salzen, bevorzugt Ethylendiamintetraessigsäure-Tetranatriumsalz,, von Disalicylaldiaminoalkanen, bevorzugt 1,2-Disalicylaldiaminopropan, von o-Hydroxygenzalaminophenolen, bevorzugt o-Hydroxybenzal-o-aminophenol, von Polyphosphaten, Carboxymethyl-mercaptobernsteinsäure oder Schiff-Basen von Salicylaldehyden. In den angeführten Beispielen der US-PS 3 121 736 wird ausschließich das teure Kaliumcarbonat als Neutralisationsmittel verwendet. Wie außerdem aus dem Vergleich der Beispiele 1 und 2 hervorgeht, wirkt sich das zugesetzt Ethylendiamintetraessigsäuretetranatriumsalz hemmend auf den Umsatz des Halogenaromaten aus, woraus zusätzliche Probleme bei der Aufarbeitung resultieren. Daneben haben eigene Versuche mit den in der US-PS 3 121 736 genannten Zusatzstoffen ergeben, daß sich diese in Gegenwart von Natriumcarbonat als Neutralisationsmittel entweder hemmend auf die Bildung von Nitrodiphenylamin auswirken oder daß sie Nebenreaktionen wie die Bildung von Nitrobenzol sehr stark begünstigen.

Es wurde nun ein Verfahren zur Herstellung von 4-Nitrodiphenylaminen der Formel (I)

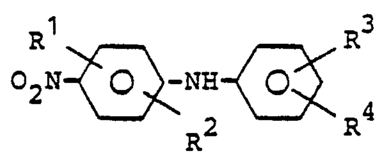

(I)

in der

$R^1$, $R^2$, $R^3$ und $R^4$ gleich oder verschieden sind und für Wasserstoff oder einen Alkylrest mit 1 bis 9 Kohlenstoffatomen stehen,

durch Umsetzung von Halogennitrobenzolen der Formel (II)

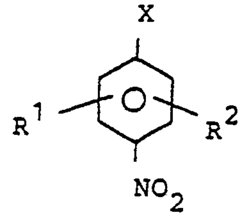

(II)

in der

X für Chlor oder Brom steht und in der

$R^1$ und $R^2$ die obengenannte Bedeutung besitzen,

mit primären aromatischen Aminen der Formel (III)

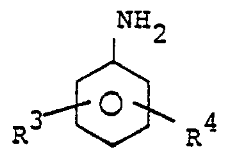

(III)

in der

$R^3$ und $R^4$ die obengenannte Bedeutung besitzen,

in Gegenwart eines Neutralisationsmitels und in Gegenwart von Kupfer oder Kupfersalzen gefunden, das dadurch gekennzeichnet ist, daß man die Umsetzung durchführt in Anwesenheit cyclischer 1,n-Diazaverbindungen mit n = 3, 4 oder 5, wobei die Stickstoffatome keinen Wasserstoff tragen und wobei mindestens ein Stickstoffatom Bestandteil einer Doppelbindung und mindestens ein Stickstoffatom Bestandteil eines Ringes ist und wobei die 1,n-Diazaverbindungen in wäßrig-alkalischer Lösung nicht zur Bildung von Anionen befähigt sind.

Als Alkylreste der Formel (I) kommen solche mit 1 bis 9, bevorzugt 1 bis 3, Kohlenstoffatomen in Frage. Genannt seien : der Methyl-, Ethyl-, n-Propyl-, iso-Propyl-, n-Butyl-, iso-Butyl-, tert.-Butyl-, n-Pentyl-, iso-Pentyl-, n-Hexyl-, n-Heptyl-, n-Octyl- und der n-Nonylrest, bevorzugt der Methyl-, Ethyl-, n-Propyl- und der iso-Propylrest.

Als cyclische 1,n-Diazaverbindungen können Imidazole oder Imidazoline der Formeln (IV) bis (VI)

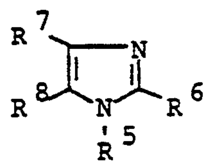

(IV)

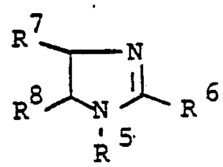

(V)

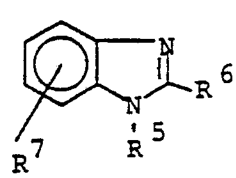

(VI)

in denen

$R^5$ Alkyl, Aryl oder Aralkyl bedeutet und

$R^6$, $R^7$, $R^8$ für Wasserstoff, Alkyl, Aryl oder Aralkyl stehen, in das erfindungsgemäße Verfahren eingesetzt werden.

Zum Beispiel können als Imidazol-Derivate eingesetzt werden.

1-Methyl-1-H-imidazol
1-Ethyl-1-H-imidazol
1-Butyl-1-H-imidazol
1-Phenyl-1-H-imidazol
1-Benzyl-1-H-imidazol
1,2-Dimethyl-1-H-imidazol
1,2,4,5-Tetramethyl-1-H-imidazol
1-Methyl-benzimidazol
1-Ethyl-2-methyl-benzimidazol
1-Butyl-4,5-dihydro-1-H-imidazol oder
1-Benzyl-2-methyl-4,5-dihydro-1-H-imidazol, bevorzugt
1-Methyl-1-H-imidazol und 1,2-Dimethyl-1-H-imidazol.

Weiterhin können als 1,n-Diazaverbindungen Pyrimidine der Formel (VII)

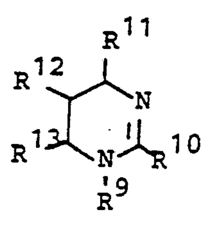

(VII)

worin

$R^9$ für Alkyl, Aryl oder Aralkyl steht und

$R^{10}$, $R^{11}$, $R^{12}$, $R^{13}$ unabhängig voneinander Wasserstoff oder Alkyl, Aryl, oder Aralkyl bedeuten oder cyclischen Amidine der Formel (VIII)

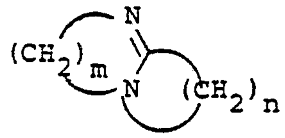

(VIII)

mit

n = 3, 4 oder 5 und m = 2, 3 oder 4,

oder Triazine der Formel (IX)

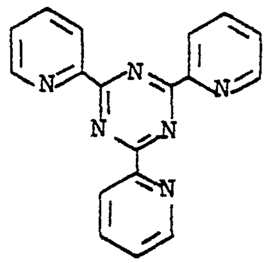

(IX)

oder Phenanthroline der Formel (X)

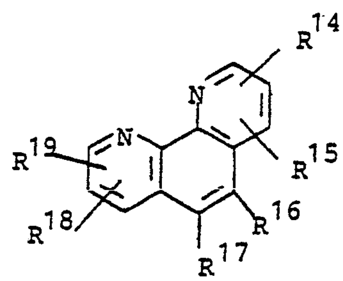

(X)

worin

$R^{14}$, $R^{15}$, $R^{16}$, $R^{17}$, $R^{18}$, $R^{19}$ gleich oder verschieden sind und für Wasserstoff, Alkyl oder Aryl stehen,

oder Dipyridine der Formel (XI)

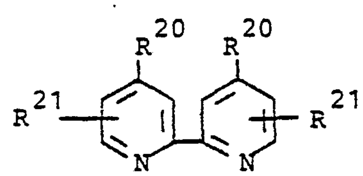

(XI)

worin

$R^{20}$ und $R^{21}$ gleich oder verschieden sind und für Wasserstoff, Alkyl oder Aryl stehen,

oder Bichinoline der Formel (XII)

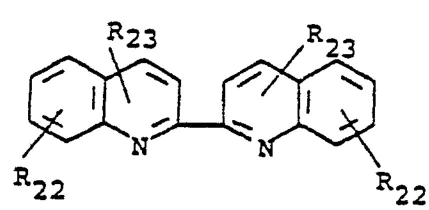

(XII)

worin

$R^{22}$ und $R^{23}$ gleich oder verschieden sind und für Wasserstoff, Alkyl oder Aryl stehen,

in das erfindungsgemäße Verfahren eingesetzt werden.

Als Alkylreste der Formeln (IV) bis (VII), (X) und (XI) kommen solche mit 1 bis 9, bevorzugt 1 bis 3 Kohlenstoffatomen in Frage. Genannt seien : der Methyl-, Ethyl-, n-Propyl-, iso-Propyl-, n-Butyl-, iso-Butyl, tert.-Butyl-, n-Pentyl-, iso-Pentyl, cyclo-Pentyl-, n-Hexyl-, cyclo-Hexyl-, n-Heptyl-, n-Octyl-, n-Nonyl-Rest, bevorzugt der Methyl-, Ethyl- und der iso-Propyl-Rest.

Als Arylreste der Formeln (IV) bis (VII) und (X) bis (XII) kommen solche mit 6 bis 14, bevorzugt 6 bis 8, Kohlenstoffatomen in Frage. Genannt seien : der Phenyl-, Tolyl-, Xylyl-, Naphthyl-Rest, bevorzugt der Phenyl-Rest.

Als Aralkylreste der Formeln (IV) bis (VII) kommen solche mit 1 bis 6, bevorzugt 1 bis 2 Kohlenstoffatomen im aliphatischen Teil und 6 bis 14, bevorzugt 6 Kohlenstoffatomen im aromatischen Teil in Frage. Genannt seien : Der Benzyl- und der Ethylphenyl-Rest, bevorzugt der Benzyl Rest.

Beipielsweise können als Pyrimidinderivate

1-Methyl-1,4,5,6-tetrahydropyrimidin
1,2-Dimethyl-1,4,5,6-tetrahydropyrimidin oder
1-Ethyl-1,4,5,6-tetrahydropyrimidin, bevorzugt
1,2-Dimethyl-1,4,5,6-tetrahydropyrimidin

als bicyclische Amidine

1,5-Diaza-bicyclo [4,3,0] nonen-(5),
1,5-Diaza-bicyclo [4,4,0] decen-(5),
1,6-Diaza-bicyclo [5,3,0] decen-(6) oder
1,8-Diaza-bicyclo [5,4,0] undecen-(7), bevorzugt
1,8-Diaza-bicyclo [5,4,0] undecen-(7)

als Triazinderivate

2,4,6-Tri-(2-pyridyl)-1,3,5-triazin,

als Phenanthroline

1,10-Phenanthrolin
2,9-Dimethyl-1,10-phenanthrolin
2,9-Dimethyl-4,7-diphenyl-1,10-phenanthrolin
4,7-Diphenyl-1,10-phenanthrolin oder
4,7-Dimethyl-1,10-phenanthrolin, bevorzugt
1,10-Phenanthrolin und 2,9-Dimethyl-1,10-pehanthrolin

als Bipyridine

2,2'-Bipyridin
4,4'-Dimethyl-2,2'-bipyridin
4,4'-Diethyl-2,2'-bipyridin
4,4',6,6'-Tetramethyl-2,2'-bipyridin oder Bichinolin, bevorzugt
2,2'-Bipyridin und Bichinolin

und als Aminopyridine

4-Dimethylaminopyridin oder
4-Pyrrolidino-amino-pyridin, bevorzugt
4-Dimethylaminopyridin

eingesetzt werden.

Selbstverständlich können die 1,n-Diazaverbindungen auch in Form ihrer Salze, beispielsweise als Hydrochloride oder im Gemisch untereinander eingesetzt werden. Die erfindungsgemäßen 1,n-Diazaverbindungen sind handelsübliche Substanzen oder können nach literaturbekannten Verfahren hergestellt werden. Die Menge der eingesetzten 1,n-Diazaverbindungen kann in weiten Grenzen schwanken. Im allgemeinen werden etwa 0,1 bis 10 Gew.-%, bevorzugt 0,5 bis 5 Gew.-%, 1,n-Diazaverbindungen, bezogen auf eingesetztes Halogen-nitrobenzol, eingesetzt.

Als Beispiel für die im erfindungsgemäßen Verfahren verwendbaren Kupferkatalysatoren seien Kupferpulver, Kupfer-(I)-jodid, Kupfer-(I)-chlorid, Kupfer-(II)-chlorid, Kupfer-(I)-bromid, Kupfer-(II)-bromid, Kupfer-(I)-cyanid, Kupfer-(I)-oxid, Kupfer-(II)-oxid, Kupfer-(II)-carbonat, basisches Kupfer-(II)-carbonat, Kupfer-(II)-sulfat, Kupfer-(II)-nitrat, Kupfer-(II)-formiat oder Kupfer-(II)-acetat genannt. Bevorzugt werden Kupfersalze schwacher Säuren, wie Kupfer-(II)-oxid, Kupfer-(II)-carbonat, basisches Kupfer-(II)-carbonat oder Kupfer-(I)-cyanid eingesetzt, wobei der Kupferkatalysator in einer Menge von wenigstens 0,1 Gew.-%, bevorzugt 0,3 bis 2 Gew.-%, bezogen auf eingesetztes Halogennitrobenzol eingesetzt wird. Die Kupferkatalysatoren können sowohl einzeln als auch im Gemisch untereinander eingesetzt werden. Sie können

auch in Form von Komplexverbindungen aus Kupfer und 1,n-Diazaverbindungen eingesetzt werden.

Durch einfache Versuche ist das für die Reaktion jeweils günstigste Gewichtsverhältnis zwischen Kupferkatalysator und 1,n-Diazaverbindung leicht zu ermitteln.

Als Halogennitrobenzole kommen für das erfindungsgemäße Verfahren Verbindungen der Formel (II)

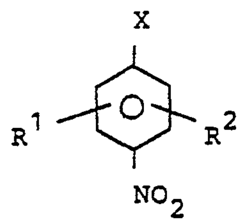

(II)

in der

$X_1$ für Chlor oder Brom steht und in der $R^1$ und $R^2$ die für Formel (I) genannte Bedeutung haben, in Betracht. Beispielsweise können folgende Verbindungen der Formel (II) eingesetzt werden : 4-Nitrochlorbenzol, 4-Nitrobrombenzol, 4-Nitro-2-methylchlorbenzol und 4-Nitro-3-methyl-chlorbenzol.

Als primäre aromatische Amine können Verbindungen der Formel (III)

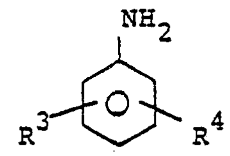

(III)

in der

$R^3$ und $R^4$ die für Formel (I) genannte Bedeutung haben, in das erfindungsgemäße Verfahren eingesetzt werden.

Zum Beispiel können folgende primäre aromatische Amine der Formel (III) eingesetzt werden : Anilin, o-Toluidin, m-Toluidin, p-Toluidin, 4-Ethylanilin, 4-Butylanilin, 4-Isopropylanilin, 3,5-Dimethylanilin und 2,4-Dimethylanilin.

Selbstverständlich können die aromatischen Amine auch in Form von Gemischen, insbesondere Isomerengemischen, eingesetzt werden. Pro Mol Halogenitrobenzol werden im allgemeinen etwa 1 bis 6 Mol, bevorzugt 1,5 bis 3 Mol, besonders 1,7 bis 2,5 Mol, des aromatischen Amins eingesetzt.

Als Neutralisationsmittel können in das erfindungsgemäße Verfahren Alkalimetallhydroxide, Alkalimetallsalze sowie Oxide von Alkalimetallen eingesetzt werden. Bevorzugt werden Alkalimetallcarbonate, insbesondere Kaliumcarbonat und/oder Natriumcarbonat eingesetzt. Das Neutralisationsmittel kann in äquivalenter Menge oder im Überschuß, bevorzugt in der 1,05- bis 1,5-fachen der äquivalenten Menge eingesetzt werden.

Durch die Verwendung der erfindungsgemäßen 1,n-Diazaverbindungen werden auch beim Einsatz von Natriumcarbonat sehr gute Ausbeuten an 4-Nitrodiphenylaminen erhalten, woraus sich wirtschaftliche und ökologische Vorteile — keine Belastung des Abwassers mit Kaliumionen — ergeben. Das Neutralisationsmittel wird vorteilhafterweise in wasserfreier und feinverteilter Form angewendet. Beispielsweise ist calcinierte Soda des Typs « Leicht » für das erfindungsgemäße Verfahren geeignet.

Die Umsetzung der Reaktanden erfolgt möglichst unter wasserfreien Bedingungen. Werden Neutralisationsmittel verwendet, bei denen während der Reaktion Wasser entsteht, so wird das Reaktionswasser vorteilhafterweise aus dem Reaktionsgemisch unter Zuhilfenahme eines Schleppmittels durch Destillation entfernt. Als Schleppmittel kommen z. B. folgende Verbindungen in Frage : Xylol, Toluol, Benzol, Chlorbenzol, Chlortoluol, Anilin und/oder Toluidin.

Das erfindungsgemäße Verfahren kan, falls gewünscht, in Gegenwart von Verdünnungsmittel, z. B. inerten organischen Kohlenwasserstoffen, beispielsweise in Gegenwart von Xylol, durchgeführt werden. Weiterhin können die aromatischen primären Amine selbst als Lösungsmittel eingesetzt werden.

Die Reaktionstemperaturen des erfindungsgemäßen Verfahrens können in weiten Bereichen schwanken. Im allgemeinen betragen sie etwa 150 bis 225 °C, vorzugsweise 185 bis 205 °C.

Die Durchführung des erfindungsgemäßen Verfahrens kann nach üblichen Methoden kontinuierlich oder diskontinuierlich erfolgen. Beispielsweise kann man bei diskontinuierlicher Verfahrensweise die Komponenten in einen mit Rührer versehenen Reaktor geben und auf Reaktionstemperatur erhitzen. Andererseits kann beispielsweise auch in einer der eigentlichen Reaktion vorgeschalteten Phase die Kupferverbindung mit der erfindungsgemäßen 1,n-Diazaverbindung, gegebenenfalls in Anwesenheit der Verbindungen der Formel (II) und/oder (III) und/oder des Schleppmittels, vorzugsweise bei einer Temperatur von 100 bis 160 °C, in Kontakt gebracht werden. Die letztgenannte Variante wird bevorzugt angewendet.

Bei der Reaktion entstehendes Wasser wird vorteilhafterweise, gegebenenfalls mit Hilfe des zusätzlichen Schleppmittels, destillativ aus dem Reaktionsgemisch entfernt. Die Reaktion wird beendet,

wenn der erwünschte Umsetzungsgrad an Halogennitrobenzol erreicht ist. Bevorzugt wird die Reaktion beendet, wenn ein Umsetzungsgrad von mindestens 96 % erreicht worden ist.

Die Aufarbeitung des Reaktionsgemisches kann ebenfalls nach verschiedenen Varianten erfolgen. In Gegenwart der erfindungsgemäßen 1,n-Diazaverbindungen ist die Teerbildung soweit zurückgedrängt, daß sich die Möglichkeit ergitb, im Reaktionsgemisch befindlihe Salze bei erhöhter Temperatur problemlos auf physikalische Weise durch Zentrifugieren oder Filtrieren abzutrennen. Nach Waschen mit warmen Xylol und Trocknen verbleibt ein hellgrauer pulvriger Feststoff. Aus dem Filtrat können nichtumgesetztes Halogennitrobenzol und primäres aromatisches Amin mit Wasserdampf ausgetrieben werden, wobei die Nitrodiphenylamine zumeist in granulierter Form anfallen. Eine andere Möglichkeit besteht darin, daß man das Filtrat im Vakuum andestilliert und das Produkt dann im Rückstand erhält oder daß man die 4-Nitrodiphenylamine weitestgehend durch Kristallisation abtrennt und/oder daß man das Filtrat mit einem Fällungsmittel, z. B. Xylol, versetzt. Die 4-Nitrodiphenyle fallen dabei in hochreiner Form an und können so direkt weiterverarbeitet werden.

Die Mutterlauge kann in den meisten Fällen rückgeführt werden, ohne daß bei erneuter Reaktion frischer Kupferkatalysator und/oder frische 1,n-Diazaverbindung eingesetzt werden muß. Gegebenenfalls wird zur Erhaltung der vollen Aktivität eine geringere als die ursprünglich eingesetzte Katalysator- und/oder 1,n-Diazaverbindung-Menge in frischer Form zugegeben. Zum Ausschleusen von Nebenprodukten wird im Bedarfsfall eine Teilmenge der anfallenden Mutterlauge abgetrennt. Das Reaktionsgemisch kann auch zur Entfernung der Salze vorzugsweise bei einer erhöhten Temperatur (85 bis 95 °C) mit der zum Lösen der Salze notwendigen Menge Wasser ausgerührt werden. Nach Phasentrennung wird die organische Schicht weiter aufgearbeitet, beispielsweise durch Wasserdampfdestillation oder durch Strippen der Bestandteile, die leichter flüchtig sind als das 4-Nitrodiphenylamin.

Wird die eingesetzte 1,n-Diazaverbindung nicht durch Wiederverwendung der bei der kristallisation anfallenden Mutterlauge rückgeführt, kann die Diazaverbindung nach gebräuchlichen Methoden in den meisten Fällen rückgewonnen werden. Dazu eignen sich z. B. destillative oder extraktive Verfahren, gegebenenfalls über die Stufe einer Salzbildung.

Nach dem erfindungsgemäßen Verfahren können 4-Nitrodiphenylamine in hervorragenden Ausbeuten und hohen Reinheiten hergestellt werden. Die Bildung von Nebenprodukten findet bei dem erfindungsgemäßen Verfahren praktisch nicht statt, so daß sich keine ökologischen Probleme ergeben. Die erfindungsgemäß eingesetzten 1,n-Diazaverbindungen wirken als starke Reaktionsbeschleuniger, wodurch der Nachteil langer Reaktionszeiten vermieden wird, was sich auf die Wirtschaftlichkeit des Verfahrens auswirkt. Außerdem können bei dem erfindungsgemäßen Verfahren billigere Neutralisationsmittel als Kaliumcarbonat verwendet werden, ohne daß dabei die Reaktion gehemmt und die Ausbeuten an 4-Nitrodiphenylamin verringert werden.

Die nach dem erfindungsgemäßen Verfahren erzielten Ausbeuten an 4-Nitrodiphenylaminen überraschen besonders im Hinblick auf J. Org. Chem. *32*, 2502, 2503, wo der Einsatz von p-Nitrobrombenzol als Halogenkomponente in einer Ullmann-Ether-Synthese beschrieben ist. Dieser Literaturstelle ist nämlich zu entnehmen, daß bei der dort beschriebenen, kupferkatalysierten Substitutionsreaktion die Ausbeute an Substitutionsprodukt durch den Zusatz von 2,2'-Dipyridin, also einer cyclischen Diazaverbindung, drastisch absinkt.

Die nach dem erfindungsgemäßen Verfahren hergestellten 4-Nitrodiphenylamine können leicht nach bekannten Verfahren zu Aminodiphenylaminen reduziert werden und sind als solche wertvolle Zwischenprodukte für die Herstellung von beispielsweise Farbstoffen oder Stabilisatoren für Kautschuk (vgl. US-PS 3163616).

Die nachfolgenden Beispiele sollen das erfindungsgemäße Verfahren verdeutlichen, ohne es jedoch auf diese Beispiele einzuschränken.

Beispiele 1 bis 5

Die nachfolgenden Beispiele 1 bis 5 der Tabelle 1 sind Vergleichsbeispiele mit Natriumcarbonat als Neutralisationsmittel.

Die Vergleichsbeispiele zeigen, daß man keine brauchbaren Ergebnisse erzielt, wenn man die in der US-PS 3 121 736 beschriebenen Komplexbildner einsetzt, und anstelle von Kaliumcarbonat Natriumcarbonat verwendet.

(Siehe Tabelle 1 Seite 8 f.)

Tabelle 1

| Beispiel Nr. | Kokatalysator | Reaktionszeit h | Gewichtsverhältnis a) Nitrobenzol : 4-Nitrochlorbenzol : 4-Nitrodiphenylamin |
|---|---|---|---|
| 1 | Glycin | 15 | 1,8 : 82,7 : 15,4 |
| 2 | o-Hydroxybenzal-o-aminophenol | 15 | 42,7 0,1 57,3 |
| 3 | 1,2-Disalicylaldiaminopropan | 15 | 34,7 0,2 65,1 |
| 4 | Nitrilotriessigsäure-trinatrium-Salz | 15 | 1,0 65,7 33,3 |
| 5 | Ethylendiamintetraessigsäuretetranatriumsalz | 24 | 2,8 52,2 45,0 |

a) hochdruckflüssigkeitschromatographisch (HPLC) ermittelt

Umsetzung von 78,7 g 4-Nitrochlorbenzol (0,5 Mol) mit 93 g Anilin (1 Mol) in Gegenwart von 38 g Natriumcarbonat leicht (0,36 Mol), 1 g CuO und 2,5 Mol % Kokatalysator (bez. auf 4-Nitrochlorbenzol). Rührapparatur mit aufgesetzter 30 cm Füllkörperkolonne und Wasserabscheider, Xylol als Wasserschlepper, Reaktionstemperatur 195 °C.

Beispiel 6 (Vergleichsbeispiel)

93 g Anilin, 2 g CuO und 60 ml Xylol werden 20 Minuten am Rückfluß und Wasserabscheider auf 150 °C erwärmt. Anschließend werden 76,3 g wasserfreies Natriumcarbonat, 157,5 g 4-Nitrochlorbenzol und 93 g Anilin zugegeben. Nach 20 h bei 195-198 °C sind 4,5 ml Wasser entstanden. Die Reaktionsmischung wird in Wasser suspendiert und alle flüchtigen Bestandteile mit Wasserdampf abgetrieben. Das rohe Nitrodiphenylamin wird durch Filtration isoliert, mit Wasser gewaschen und getrocknet.

91,5 g 4-Nitrodiphenylamin, Gehalt (HPLC) 80,8 %, Ausbeute 34,5 % der Theorie, bezogen auf eingesetztes p-Nitrochlorbenzol.

Beispiel 7

93 g Anilin, 3,8 g 1,8-Diazabicyclo [5,4,0] undec-7-en, 2 g Kupfer-(II)-oxid und 40 ml Xylol werden zusammen 20 Minuten am Wasserabscheider auf 150 °C erhitzt. Anschließend werden 157,5 g 4-Nitrochlorbenzol, 93 g Anilin und 76 g calcinierte Soda (leicht) zugegeben. Nach 12 Stunden bei 193-196 °C haben sich 10 ml Wasser im Wasserabscheider gesammelt. Das Gewichtsverhältnis Nitrobenzol : 4-Nitrochlorbenzol : 4-Nitrodiphenylamin beträgt 1,5 : 2,0 : 96,5 im Reaktionsgemisch. Das Reaktionsgemisch wird auf 95 °C abgekühlt, abfiltriert, der Filterkuchen mit 100 ml heißem Xylol gewaschen. Das Filtrat wird mit Wasser versetzt und unumgesetztes 4-Nitrochlorbenzol und Anilin mit Wasserdampf abgetrieben, wobei das 4-Nitrodiphenylamin granuliert. Es wird durch Filtration isoliert, mit heißem Wasser gewaschen und getrocknet : 210 g 4-Nitrodiphenylamin, Gehalt (HPLC) 91,3 %, Ausbeute 89,6 % der Theorie, bezogen auf eingesetztes p-Nitrochlorbenzol.

Beispiel 8

Beispiel 8 wurde analog Beispiel 7 durchgeführt, jedoch mit 100 g Kaliumcarbonat anstelle von Soda. Reaktionszeit 7 Stunden, Aufarbeitung wie in Beispiel 7 beschrieben : 214 g 4-Nitrodiphenylamin, Gehalt (HPLC) 92,6 %, Ausbeute 92,6 % der Theorie.

Beispiele 9 bis 16

In der nachfolgenden Tabelle 2 sind die Beispiele 9 bis 16 aufgeführt.

(Siehe Tabelle 2 Seite 10 f.)

Tabelle 2

| Beispiel Nr. | 1,n-Diazaverbindung | Reaktionszeit [h] | Neutralisationsmittel | Gew.-Verhältnis 4-Nitrochlorbenzol : 4-Nitrodiphenylamin im Reaktionsgemisch | 4-Nitrodiphenylamin Ausbeute [% d. Th.] bez. auf eingesetztes 4-Nitrochlorbenzol |
|---|---|---|---|---|---|
| 9 | 1,10-Phenanthrolin | 10 | $Na_2CO_3$ | 0,5 : 95,5 | 90,8 |
| 10 | 2,2'-Bipyridin | 12 | $Na_2CO_3$ | 2,3 : 97,7 | 87,6 |
| 11 | 1,2-Dimethyl-1,4,5,6-tetrahydropyrimidin | 15 | $Na_2CO_3$ | 4,5 : 95,5 | 86,8 |
| 12 | 1,2-Dimethyl-imidazol | 15 | $Na_2CO_3$ | 0,9 : 99,1 | 80,9 |
| 13 | 1,8-Diaza-bicyclo [5,4,0] undec-7-en | 10 | $NaHCO_3$ | 1,3 : 98,7 | 85,6 |
| 14 | 4-Dimethylamino-pyridin | 12 | $Na_2CO_3$ | 0,4 : 99,6 | 84,4 |
| 15 | 2,2'-Bipyridin | 8 | $K_2CO_3$ | 1,5 : 98,5 | 88,5 |
| 16 | 1,2-Dimethyl-imidazol | 6 | $K_2CO_3$ | 3,5 : 96,5 | 85,2 |

186 g (2 Mol) Anilin, 157,5 g (1 Mol) 4-Nitrochlorbenzol, 2 g CuO, 1,42 Äquivalente Neutralisationsmittel, 0,025 Mol 1,n-Diazaverbindung ; Durchführung und Aufarbeitung wie im Beispiel 7 beschrieben.

Beispiel 17

186 g Anilin, 60 ml Xylol, 4,5 g 1,10-Phenanthrolin, 157,5 g 4-Nitrochlorbenzol, 2 g CuO und 76,3 g Kaliumcarbonate werden 7 Stunden am Wasserabscheider auf 195 °C erhitzt. Das Reaktionsgemisch wird auf 96 °C abgekühlt. Nach Abtrennen der ausgefallenen Salze durch Filtration wird der Filterkuchen mit 200 ml heißem Xylol ausgewaschen. Filtrat und Waschflüssigkeit werden vereinigt, nach Abkühlen auf 0 °C kristallisieren 127 g 4-Nitrodiphenylamin, Fp. 128-130 °C aus und werden durch Filtration abgetrennt. Die hierbei anfallende Mutterlauge wird mit 93 g Anilin, 157,5 g p-Nitrochlorbenzol und 76,3 g Kaliumcarbonat aufgefüllt, destillativ vom überschüssigen Xylol befreit und 11 Stunden bei 195 °C am Wasserabscheider erhitzt. Nach erneutem Abfiltrieren von den Salzen wird durch Wasserdampfdestillation wie in Beispiel 7 beschrieben aufgearbeitet. Man erhält 277 g 4-Nitrodiphenylamin vom Fp. 107-111 °C.

Beispiel 18

121 g 3,5-Xylidin, 30 ml Xylol, 2,2 g 1,10-Phenanthrolin und 1 g CuO werden am Wasserabscheider 30 Minuten auf 155 °C erhitzt. Nach Zugabe von 78,7 g 4-Nitrochlorbenzol und 38,1 g Natriumcarbonat wird 7 Stunden bei 193-195 °C am Wasserabscheider gekocht, dabei entstehen 5,2 ml Wasser. Die Aufarbeitung erfolgt wie in Beispiel 7 beschrieben. Man erhält 110 g 3,5-Dimethyl-4'-nitrodiphenylamin vom Fp. 179-181 °C.

Beispiel 19

Analog Beispiel 18, jedoch werden anstelle von 3,5-Xylidin 107 g p-Toluidin eingesetzt. Die Reaktionszeit beträgt 8 Stunden. Man erhält 114 g 4'-Methyl-4-nitrodiphenylamin vom Fp. 120-122 °C.

Beispiel 20

Analog Beispiel 18, jedoch werden anstelle von 3,5-Xylidin 107 g o-Toluidin eingesetzt. Die Reaktionszeit beträgt 12 Stunden. Man erhält 107 g rohes 2'-Methyl-4-nitro-diphenylamin als Rückstand bei der Wasserdampfdestillation.

Beispiel 21

186 g Anilin, 30 ml Xylol, 4,5 g 1,10-Phenanthrolin und 2 g CuO werden am Wasserabscheider 20 Minuten auf 150-155 °C erhitzt. Nach Zugabe von 157,5 g 4-Nitrochlorbenzol und 76 g Natriumcarbonat wird das Reaktionsgemisch 9 Stunden am Wasserabscheider bei 193-195 °C gekocht, wobei 9,9 ml Wasser anfallen. Das Verhältnis Nitrobenzol : 4-Nitrochlorbenzol : 4-Nitrodiphenylamin beträgt 2,8 : 2,7 : 94,5 Gew.-Teile. Nach Abkühlen auf 110 °C wird von den Salzen abfiltriert und der Filterkuchen mit 50 ml 90 °C warmen Xylol gewaschen. Filtrat und Waschflüssigkeit werden vereinigt und im Vakuum (15 mbar, Heizbadtemperatur bis 130 °C) andestilliert. Der Destillationsrückstand wird noch warm mit 160 ml Xylol versetzt und unter Rühren auf 5 °C abgekühlt. Das auskristallisierte Produkt wird scharf abfiltriert, der Filterkuchen mit 50 ml eiskaltem Xylol gewaschen und getrocknet : 181 g feinkristallines 4-Nitrodiphenylamin vom Festpunkt 122-125 °C. Das anfallende Filtrat wird destillativ vom überschüssigen Xylol befreit und nach Zugabe von 206 g Anilin, 157,5 g 4-Nitrochlorbenzol und 76 g Natriumcarbonat rückgeführt.

Nach 11 Stunden bei 193-195 °C sind 10,2 ml Wasser entstanden. Der 4-Nitrochlorbenzol-Umsatz beträgt 97 %. Die Isolierung des 4-Nitrodiphenylamins erfolgt wie vorstehend beschrieben durch Kristallisation. Man erhält nach Trocknen 177 g kristallines 4-Nitrodiphenylamin vom Schmelzpunkt 119-120 °C.

Nach Versetzen mit 4 g kristallwasserhaltigem Natriumsulfid wird die Mutterlauge filtriert und anschließend dreimal mit verdünnter Salzsäure extrahiert. Die wäßrigen Phasen werden vereinigt, alkalisch gestellt und mit Dichlormethan extrahiert. Der nach Entfernen des Lösungsmittels erhaltene Extrakt enthält 1,10-Phenanthrolin.

Beispiel 22

Zu einer heißen Lösung von 3,9 g 1,10-Phenanthrolin in 100 ml Wasser gibt man 1,7 g $CuCl_2 \times 2\ H_2O$ in Form einer konzentrierten wäßrigen Lösung. Nach Erkalten wird der auskristallisierte grüne Kupferkomplex durch Filtration isoliert und bis zur Gewichtskonstanz über $P_4O_{10}$ getrocknet.

93 g Anilin, 3,5 g des wie vorstehend beschrieben erhaltenen Kupferkomplexes, 78,5 g 4-Nitrochlorbenzol und 38 g calcinierte Soda werden nach Zugabe von 30 ml Xylol am Wasserabscheider auf 193-196 °C erhitzt.

Nach 10 Stunden wird 4,9 ml Wasser abgeschieden. Das Gewichtsverhältnis 4-Nitrodiphenylamin : p-Nitrochlorbenzol im Reaktionsgemisch beträgt 97,3 : 2,7.

**Patentansprüche**

1. Verfahren zur Herstellung von 4-Nitrodiphenylaminen der Formel

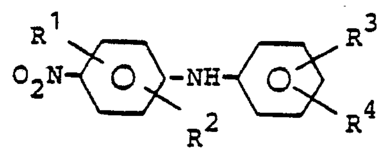

in der

$R^1$, $R^2$, $R^3$ und $R^4$ gleich oder verschieden sind und für Wasserstoff oder einen Alkylrest mit 1 bis 9 Kohlenstoffatomen stehen,

durch Umsetzung von Halogennitrobenzolen der Formel

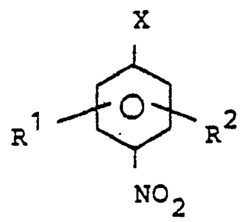

in der

$X_1$ für Chlor oder Brom steht und in der

$R^1$ und $R^2$ die obengenannte Bedeutung besitzen,

mit primären aromatischen Aminen der Formel

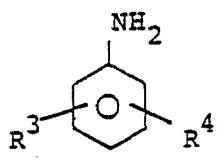

in der

$R^3$ und $R^4$ die obengenannte Bedeutung besitzen,

in Gegenwart eines Neutralisationsmittels und in Gegenwart von Kupfer oder Kupferverbindungen, dadurch gekennzeichnet, daß man die Umsetzung durchführt in Anwesenheit cyclischer 1,n-Diazaverbindungen mit n = 3, 4 oder 5, wobei die Stickstoffatome keinen Wasserstoff tragen und wobei mindestens ein Stickstoffatom Bestandteil einer Doppelbindung und mindestens ein Stickstoffatom Bestandteil eines Ringes ist und wobei die 1,n-Diazaverbindungen in wäßrig-alkalischer Lösung nicht zur Bildung von Anionen befähigt sind.

2. Verfahren nach Anspruch 1, dadurch gekennzeichnet, daß man als cyclische 1,n-Diazaverbindungen Imidazole, Imidazoline, Pyrimidine, bicyclische Amidine, Triazine, Phenantroline, Dipyridine und/oder Bichinoline einsetzt.

3. Verfahren nach Ansprüchen 1 und 2, dadurch gekennzeichnet, daß man als 1,n-Diazaverbindungen eine oder mehrere Verbindungen aus der Gruppe 1-Methyl-1-H-imidazol, 1-Ethyl-1-H-imidazol, 1-Butyl-1-H-imidazol, 1-Phenyl-1-H-imidazol, 1-Benzyl-1-H-imidazol, 1,2-Dimethyl-1-H-imidazol, 1,2,4,5-Tetramethyl-1-H-imidazol, 1-Methyl-benzimidazol, 1-Ethyl-2-methyl-benzimidazol, 1-Butyl-4,5-dihydro-1-H-imidazol, 1-Benzyl-2-methyl-4,5-dihydro-1-H-imidazol, 1-Methyl-1,4,5,6-tetrahydropyrimidin, 1,-2-dimethyl 1,4,5,6-tetrahydropyrimidin, 1-Ethyl-1,4,5,6-tetrahydropyrimidin, 1,5-Diaza-bicyclo [4,3,0]-nonen-(5), 1,5-Diaza-bicyclo-[4,4,0]-decen-(5), 1,6-Diaza-bicyclo [5,3,0]-decen-(6), 1,8-Diaza-bicyclo [5,4,0]-undecen-(7), 2,4,6-Tri-(2-pyridyl)-1,3,5-triazin, 1,10-Phenanthrolin, 2,9-Dimethyl-1,10-phenanthrolin, 2,9-Dimethyl-4,7-diphenyl-1,10-phenanthrolin, 4,7-Diphenyl-1,10-phenanthrolin, 4,7-Dimethyl-1,10-phenanthrolin, 2,2'-Bipyridin, 4,4'-Dimethyl-2,2'-bipyridin, 4,4'-Diethyl-2,2'-bipyridin, 4,4',6,6'-Tetramethyl-2,2'-bipyridin, Bichinolin, 4-Dimethylamino-pyridin oder 4-Pyrrolidino-amino-pyridin einsetzt.

4. Verfahren nach Ansprüchen 1 bis 2, dadurch gekennzeichnet, daß man als 1,n-Diazaverbindungen eine oder mehrere Verbindungen aus der Gruppe 1-Methyl-1-H-imidazol, 1,2-Dimethyl-1-H-imidazol, 1,2-Dimethyl-1,4,5,6-tetrahydropyrimidin, 1,8-Diaza-bicyclo [5,4,0]-undecen-(7), 1,10-Phenanthrolin, 2,9-Dimethyl-1,10-phenanthrolin, 2,2'-Bipyridin, Bichinolin oder 4-Dimethylaminopyridin einsetzt.

5. Verfahren nach Ansprüchen 1 bis 4, dadurch gekennzeichnet, daß man die 1,n-Diazaverbindungen in Mengen von 0,1 bis 10 Gew.-%, bezogen auf eingesetztes Halogennitrobenzol, einsetzt.

6. Verfahren nach Ansprüchen 1 bis 4, dadurch gekennzeichnet, daß man die 1,n-Diazaverbindungen in Mengen von 0,5 bis 5 Gew.-%, bezogen auf eingesetztes Halogennitrobenzol, einsetzt.

**Claims**

1. Process for the preparation of 4-nitrodiphenylamines of the formula

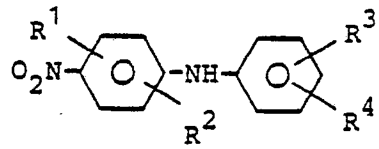

in which

$R^1$, $R^2$, $R^3$ und $R^4$ are identical or different and represent hydrogen or an alkyl radical having 1 to 9 carbon atoms,

by the reaction of halogenonitrobenzenes of the formula

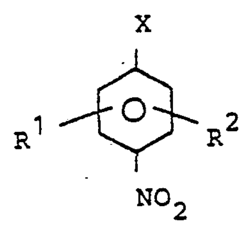

in which

$X_1$ represents chlorine or bromine and

$R^1$ and $R^2$ have the abovementioned meaning,

with primary aromatic amines of the formula

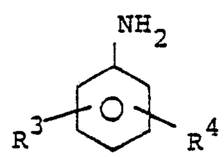

in which

$R^3$ and $R^4$ have the abovementioned meaning,

in the presence of a neutralising agent and in the presence of copper or copper compounds, characterised in that the reaction is carried out in the presence of cyclic 1,n-diaza compounds with n = 3, 4 or 5, wherein the nitrogen atoms do not carry any hydrogen and at least one nitrogen atom is a constituent of a double bond and at least one nitrogen atom is a constituent of a ring, and the 1,n-diaza compounds are not capable of forming anions in aqueous-alkaline solution.

2. Process according to Claim 1, characterised in that imidazoles, imidazolines, pyrimidines, bicyclic amidines, triazines, phenantrolines, dipyridines and/or biquinolines are employed as the cyclic 1,n-diaza compounds.

3. Process according to Claims 1 and 2, characterised in that one or more compounds from the group comprising 1-methyl-1-H-imidazole, 1-ethyl-1-H-imidazole, 1-butyl-1-H-imidazole, 1-phenyl-1-H-imidazole, 1-benzyl-1-H-imidazole, 1,2-dimethyl-1-H-imidazole, 1,2,4,5-tetramethyl-1-H-imidazole, 1-methyl-benzimidazole, 1-ethyl-2-methyl-benzimidazole, 1-butyl-4,5-dihydro-1-H-imidazole, 1-benzyl-2-methyl-4,5-dihydro-1-H-imidazole, 1-methyl-1,4,5,6-tetrahydropyrimidine, 1,2-dimethyl-1,4,5,6-tetrahydropyrimidine, 1-ethyl-1,4,5,6-tetrahydropyrimidine, 1,5-diaza-bicyclo [4,3,0] non-5-ene, 1,5-diaza-bicyclo [4,4,0] dec-5-ene, 1,6-diaza-bicyclo [5,3,0]-dec-6-ene, 1,8-diaza-bicyclo [5,4,0] undec-7-ene, 2,4,6-tri-(pyrid-2-yl)-1,3,5-triazine, 1,10-phenanthroline, 2,9-dimethyl-1,10-phenanthroline, 2,9-dimethyl-4,7-diphenyl-1,10-phenanthroline, 4,7-diphenyl-1,10-phenanthroline, 4,7-dimethyl-1,10-phenanthroline, 2,2'-bipyridine, 4,4'-dimethyl-2,2'-bipyridine, 4,4'-diethyl-2,2'-bipyridine, 4,4',6,6'-tetramethyl-2,2'-bipyridine, biquinoline, 4-dimethylaminopyridine and 4-pyrrolidino-aminopyridine are employed as the 1,n-diaza compounds.

4. Process according to Claims 1 to 2, characterised in that one or more compounds from the group

comprising 1-methyl-1-H-imidazole, 1,2-dimethyl-1-H-imidazole, 1,2-dimethyl-1,4,5,6-tetrahydropyrimidine, 1,8-diaza-bicyclo [5,4,0] undec-7-ene, 1,10-phenanthroline, 2,9-dimethyl-1,10-phenanthroline, 2,2′-bipyridine, biquinoline and 4-dimethylaminopyridine are employed as the 1,n-diaza compounds.

5. Process according to Claims 1 to 4, characterised in that the 1,n-diaza compounds are employed in amounts of from 0.1 to 10 % by weight, relative to halogenonitrobenzene employed.

6. Process according to Claims 1 to 4, characterised in that the 1,n-diaza compounds are employed in amounts of from 0.5 to 5 % by weight, relative to halogenonitrobenzene employed.

**Revendications**

1. Procédé de préparation de 4-nitrodiphénylamines de formule

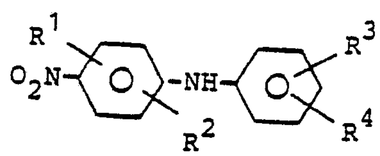

dans laquelle

$R^1$, $R^2$, $R^3$ et $R^4$, ayant des significations identiques ou différentes, représentent l'hydrogène ou un groupe alkyle en $C_1$-$C_9$,

par réaction d'halogénonitrobenzènes de formule

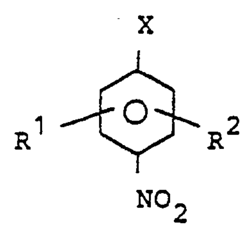

dans laquelle

X représente le chlore ou le brome et

$R^1$ et $R^2$ ont les significations indiquées ci-dessus,

avec des amines aromatiques primaires de formule

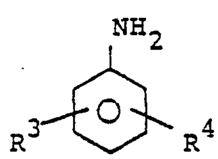

dans laquelle

$R^3$ et $R^4$ ont les significations indiquées ci-dessus,

en présence d'un agent neutralisant et en présence de cuivre ou de composés de cuivre, caractérisé en ce que l'on effectue la réaction en présence de composés cycliques en 1,n-diaza dans lesquels n = 3, 4 ou 5, les atomes d'azote ne portant pas d'hydrogène et au moins un atome d'azote étant constituant d'une double liaison et au moins un atome d'azote constituant d'un cycle ; les composés en 1,n-diaza n'étant pas capables de former des anions en solution aqueuse alcaline.

2. Procédé selon la revendication 1, caractérisé en ce que l'on utilise en tant que composés cycliques en 1,n-diaza des imidazoles, des imidazolines, des pyrimidines, des amidines bicycliques, des triazines, des phénanthrolines, des dipyridines et/ou des biquinoléines.

3. Procédé selon les revendications 1 et 2, caractérisé en ce que l'on utilise en tant que composés en 1,n-diaza un ou plusieurs composés du groupe formé par le 1-méthyl-1-H-imidazole, le 1-éthyl-1-H-imidazole, le 1-butyl-1-H-imidazole, le 1-phényl-1-H-imidazole, le 1-benzyl-1-H-imidazole, le 1,2-diméthyl-1-H-imidazole, le 1,2,4,5-tétraméthyl-1-H-imidazole, le 1-méthyl-benzimidazole, le 1-éthyl-2-méthyl-benzimidazole, le 1-butyl-4,5-dihydro-1-H-imidazole, le 1-benzyl-2-méthyl-4,5-dihydro-1-H-imidazole, la 1-méthyl-1,4,5,6-tétrahydropyrimidine, la 1,2-diméthyl-1,4,5,6-tétrahydropyrimidine, la 1-éthyl-1,4,5,6-tétrahydropyrimidine, le 1,5-diaza-bicyclo [4,3,0]-nonène-(5), le 1,5-diaza-bicyclo [4,4,0] décène-(5), le 1,6-diaza-bicyclo [5,3,0] décène-(6), le 1,8-diaza-bicyclo [5,4,0] undécène-(7), la 2,4,6-tri-(2-pyridyl)-1,3,5-triazine, la 1,10-phénanthroline, la 2,9-diméthyl-1,10-phénanthroline, la 2,9-diméthyl-4,7-diphényl-1,10-phénanthroline, la 4,7-diméthyl-1,10-phénanthroline, la 2,2′-bipyridine, la 4,4′-diméthyl-2,2′-bipyridine,

la 4,4′-diéthyl-2,2′-bipyridine, la 4,4′,6,6′-tétraméthyl-2,2′-bipyridine, la biquinoléine, la 4-diméthylamino-pyridine, ou la 4-pyrrolidino-amino-pyridine.

4. Procédé selon les revendications 1 et 2, caractérisé en ce que l'on utilise en tant que composés en 1,n-diaza un ou plusieurs composés du groupe formé par le 1-méthyl-1-H-imidazole, le 1,2-diméthyl-1-H-imidazole, la 1,2-diméthyl-1,4,5,6-tétrahydropyrimidine, le 1,8-diaza-bicyclo [5,4,0] undécène-(7), la 1,10-phénanthroline, la 2,9-diméthyl-1,10-phénanthroline, la 2,2′-bipyridine, la biquinoléine ou la 4-diméthylaminopyridine.

5. Procédé selon les revendications 1 à 4, caractérisé en ce que l'on utilise les composés en 1,n-diaza en quantités de 0,1 à 10 % en poids par rapport à l'halogénonitrobenzène mis en œuvre.

6. Procédé selon les revendications 1 à 4, caractérisé en ce que l'on utilise les composés en 1,n-diaza en quantités de 0,5 à 5 % en poids par rapport à l'halogénonitrobenzène mis en œuvre.